Europäisches Patentamt

European Patent Office

Office européen des brevets

(19)

(11) Veröffentlichungsnummer : **0 151 371**
**B1**

(12) # EUROPÄISCHE PATENTSCHRIFT

(45) Veröffentlichungstag der Patentschrift :
06.05.87

(51) Int. Cl.⁴ : **A 61 M   5/14**

(21) Anmeldenummer : **84810655.5**

(22) Anmeldetag : **21.12.84**

(54) **Wirkstoffabgabevorrichtung für parenterales Applikationssystem und damit ausgestattetes parenterales Applikationssystem.**

(30) Priorität : **03.02.84 CH 510/84**

(43) Veröffentlichungstag der Anmeldung :
**14.08.85 Patentblatt 85/33**

(45) Bekanntmachung des Hinweises auf die Patenterteilung : **06.05.87 Patentblatt 87/19**

(84) Benannte Vertragsstaaten :
**BE CH DE FR GB IT LI NL SE**

(56) Entgegenhaltungen :
**EP-A- 0 059 694**
**GB-A- 2 104 862**

(73) Patentinhaber : **CIBA-GEIGY AG**
**Klybeckstrasse 141**
**CH-4002 Basel (CH)**

(72) Erfinder : **Geller, Leo, Dr.**
**Rudolf Wackernagel-Strasse 14**
**CH-4125 Riehen (CH)**

## Beschreibung

Die Erfindung betrifft eine Wirkstoffabgabevorrichtung zum Einfügen in die Flüssigkeitsleitung eines parenteralen Applikationssystems gemäss dem ersten Teil des Patentanspruchs 1 sowie ein mit einer solchen Wirkstoffabgabevorrichtung ausgestattetes Applikationssystem gemäss dem ersten Teil des Patentanspruchs 9.

Die intravenöse Verabreichung von Flüssigkeiten wie Blut, Blutersatz und anderen Infusionslösungen ist ein wichtiger Bestandteil der ärztlichen Behandlung von Patienten. In vielen Fällen wird ausserdem gleichzeitig bzw. zusammen mit der Infusionslösung auch noch irgendein anderes Medikament intravenös verabreicht, wofür eine Reihe von verschiedenen Systemen existieren. So ist z. B. bei einem aus der DE-A-3 228 595 (entsprechend den US-Patentanmeldungen Ser. Nos. 289 082, 312 491, 310 047 und 325 206) bekannten System dieser Art in die Infusionsleitung oder parallel zu einem Abschnitt derselben ein Behälter eingeschleift, in welchem der zu verabreichende Wirkstoff in einer besonderen Formulierung (Osmose-System) enthalten ist, welche eine über einen längeren Zeitraum andauernde Wirkstoffabgabe an die Infusionslösung gewährleistet. Der Nachteil dieses Systems besteht in erster Linie darin, dass der Wirkstoff unbedingt in einer speziellen Formulierung vorliegen muss und dass ausserdem das Einschleifen des Behälters in die Infusionsleitung, insbesondere wenn dies mittels einer By-pass-Leitung erfolgen soll, relativ umständlich ist. Ein weiterer Nachteil des bekannten Systems besteht darin, dass es relativ schwierig ist, allenfalls im Behälter vorhandene Luft aus diesem zu entfernen.

Ein ähnliches System mit denselben Nachteilen ist auch aus der GB-A-2 104 862 bekannt.

In der prioritätsälteren EP-Patentanmeldung EP-A-0 100 296 (entsprechend US-Pat. Anmeldung Ser. No. 06/500 744 vom 3. Juni 1983) ist eine unmittelbar in die Infusionsleitung einzufügende Wirkstoffabgabevorrichtung in Form eines Bechers beschrieben, der in seinem Inneren einen Haupt- und einen Nebenströmungsweg für die Infusionsflüssigkeit besitzt, wobei der Nebenströmungsweg einen in Gebrauch von unten nach oben durchflossenen Abschnitt aufweist und der Wirkstoff in diesem Abschnitt auf einem Träger angeordnet ist. Dieses System bringt bereits erhebliche Vorteile gegenüber den bekannten Systemen, ist jedoch ebenfalls noch verbesserungsfähig.

Aus der EP-A-0 059 694 ist ferner eine Wirkstoffabgabevorrichtung für parenterale Systeme beschrieben, bei der in einem von der zu applizierenden Flüssigkeit durchströmten rohrförmigen Behältnis eine oben offene Staukammer vorgesehen ist, welche den Wirkstoff in Form von Granulat oder dgl. enthält. Die Flüssigkeit gelangt zuerst in die Staukammer, fliesst über deren Rand über und von da über ein Filter zum Auslass des Behältnisses. Der in der Staukammer befindliche Wirkstoff geht nach und nach in dem in der Staukammer enthaltenen und sich laufend erneuernden Flüssigkeitsvolumen in Lösung und mischt sich auf diese Weise der durch das System strömenden Flüssigkeit bei.

Bei dieser bekannten Wirkstoffagabevorrichtung ist die Wirkstofffreisetzung vom Durchfluss abhängig und empfindlich auf Druckänderungen innerhalb des parenteralen Systems. Ausserdem ist bei dieser Vorrichtung nicht ausreichend sichergestellt, dass der Wirkstoff auch tatsächlich in der vorbestimmten Weise in das überlaufende Flüssigkeitsquantum gelangt. Die in vielen Fällen erforderliche zeitliche Konstanz der Wirkstoffabgabe ist daher mit dieser Vorrichtung nicht gewährleistet.

Durch die vorliegende Erfindung soll nun eine Wirkstoffabgabevorrichtung der eingangs definierten Art dahingehend verbessert werden, dass sie einerseits eine möglichst gute, gleichmässige und kontrollierte Abgabe des Wirkstoffs gewährleistet, wobei der Wirkstoff nicht in einer speziellen Formulierung vorzuliegen braucht, und dass sie anderseits konstruktiv einfach sowie einfach und leicht in der Handhabung und Bedienung ist.

Die erfindungsgemässe Wirkstoffabgabevorrichtung und das damit ausgestattete erfindungsgemässe parenterale Applikationssystem sind in den Patentansprüchen 1 und 9 beschrieben. Vorteilhafte Weiterbildungen ergeben sich aus den abhängigen Ansprüchen.

Bei der erfindungsgemässen Wirkstoffabgabevorrichtung ist also die Staukammer einerseits mit einer in den als Luftraum wirkenden unteren Teil des Behälters ausmündenden Kapillare versehen und andererseits praktisch frei im Behältnis aufgehängt. Durch diese Ausbildung und Anordnung wird gewährleistet, dass der Wirkstoff der Flüssigkeit mit hoher zeitlicher Konstanz zugeführt wird, wobei der Druckausgleich im System gewährleistet ist und Druckschwankungen daher ohne Einfluss bleiben.

Im folgenden wird die Erfindung anhand der Zeichnung näher erläutert. Es zeigen :

Figur 1 eine Gesamtansicht eines erfindungsgemässen parenteralen Applikationssystems,

Figur 2 einen Längsschnitt durch eine erfindungsgemässe Wirkstoffabgabevorrichtung,

Figur 3 einen Querschnitt nach der Linie III-III der Fig. 2 und

Figur 4 eine grössere Darstellung eines Details aus Fig. 2.

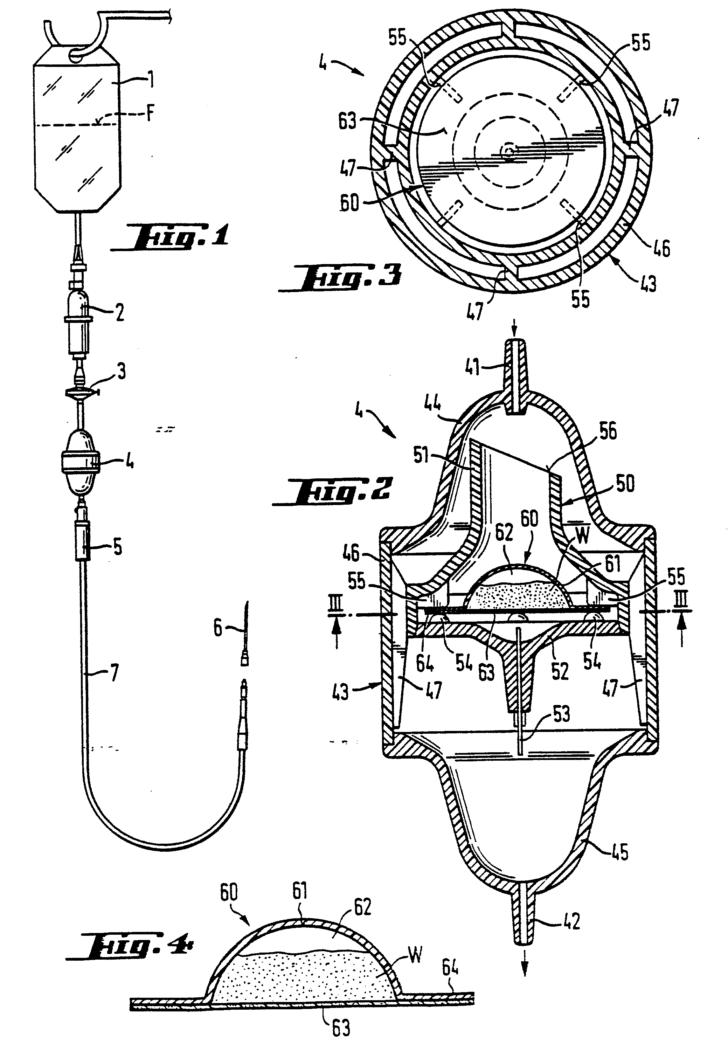

Das Applikationssystem umfasst gemäss Fig. 1 einen an einem nicht bezeichneten Haken aufgehängten Vorratsbehälter 1 für Infusionsflüssigkeit F, einen Tropfenzähler 2, einen Durchflussregulator 3, eine Wirkstoffabgabevorrichtung 4, ein Sterilfilter 5 und eine Infusionsnadel 6 sowie eine die Nadel mit dem Sterilfilter verbindende Leitung 7. Mit Ausnahme der Wirkstoffabgabevorrichtung 4 sind alle Teile des dargestellten Systems konventionell, sodass sich die nachstehenden Erläuterungen auf die erfindungsgemässe Wirkstoffabgabevorrichtung beschränken können.

Die Wirkstoffabgabevorrichtung 4 besteht im wesentlichen aus einem mit Ausnahme eines Einlass-Anschlusses 41 und eines Auslass-Anschlusses 42 allseits geschlossenen Behälter 43, in dessen Inneren eine Staukammer 50 vorgesehen ist, in welcher sich wiederum ein Behältnis 60 mit dem abzugebenden Wirkstoff W befindet.

Der Behälter 43 ist aus drei Kunststoff-Teilen zusammengesetzt, und zwar aus einem Deckelteil 44, einem Bodenteil 45 und einem im wesentlichen zylindrischen Mittelteil 46. Letzterer weist an seiner Innenseite radial einwärts vorspringende Wandrippen 47 auf, welche den kaminartig ausgebildeten Oberteil 51 der Staukammer 50 tragen. Der Unterteil oder Boden 52 der Staukammer ist ein separates Teil und mit dem Oberteil verklebt oder verschweisst.

Den Boden 52 der Staukammer 50 ist in der Mitte sumpfartig vertieft. In diese vertiefte Stelle ist eine Kapillare 53 eingesetzt, welche den Boden durchbricht und eine als Restriktion wirkende Austrittsöffnung für in der Staukammer 50 befindliche Infusionsflüssigkeit bildet.

Das Behältnis 60 für den Wirkstoff W besteht aus einem in Form einer Mulde oder eines Napfes tiefgezogenen Kunststoffteil 61 und einer den Hohlraum 62 verschliessenden Membrane 63. Der Wirkstoff W befindet sich im Hohlraum 62. Das Wirkstoffbehältnis 60 liegt mit seiner flachen durch die Membrane 63 abgeschlossenen Seite nach unten mittels seines flanschartigen Randes 64 auf am Boden 52 der Staukammer 50 angeordneten bzw. ausgebildeten Stützstegen 54 auf und wird durch am Oberteil 51 der Staukammer 50 ausgebildete radiale Halterippen 55 in seiner Position fixiert.

Die Funktionsweise der gezeigten Vorrichtung ist wie folgt :

Infusionsflüssigkeit F tritt durch den Einlass 41 in den Behälter 43 ein und gelangt direkt in die Staukammer 50. Wegen der restriktiven Wirkung der Kapillare 53 kann die Flüssigkeit nicht im selben Masse aus der Staukammer abfliessen, wie sie in diese eintritt, und füllt daher die Staukammer auf. Nach dem Erreichen des als Ueberlauf wirkenden Oeffnungsrands 56 der Staukammer 50 läuft die Flüssigkeit über und strömt seitlich neben der Staukammer in den Zwischenräumen zwischen der Kammer und der Behälterinnenwand nach unten zum Auslass.

Die das Wirkstoffbehältnis 60 unten abschliessende Membrane 63 ist für die jeweilige Infusionsflüssigkeit durchlässig ausgebildet. Die Flüssigkeit kann daher in das Behältnis 60 eindringen und darin enthaltenen Wirkstoff W lösen. Diese Wirkstofflösung fliesst dann durch die Kapillare 53 ab und mischt sich dem aussen an der Staukammer 50 vorbeifliessenden Hauptflüssigkeitsstrom bei. Die Konzentration des Wirkstoffs im (Gesamt-) Infusionsflüssigkeitsstrom hängt vom durch die Kapillare 53 bestimmten By-Pass-Verhältnis der beiden Teilflüssigkeitsströme (durch die und neben der Staukammer) sowie von der pro Zeiteinheit in Lösung gehenden Wirkstoffmenge (Löslichkeit, wirksame Oberfläche, Membraneigenschaften) ab. Durch geeignete Wahl der Kapillare und der Membran bzw. Abstimmung dieser Teile auf die Lösungseigenschaften des Wirkstoffs in der Infusionsflüssigkeit kann praktisch jede gewünschte Wirkstoffdosierung (und Dauer der Dosierung bis zum vollständigen Verbrauch des Wirkstoffs) erreicht werden. Insbesondere ist es mit der gezeigten Vorrichtung möglich, eine zeitlich konstante Wirkstoffabgabe zu erzielen. Dazu trägt u. a. auch bei, dass das Wirkstoffbehältnis 60 mittels einer ebenen Membrane abgeschlossen und mit dieser Membrane nach unten (horizontale Membranfläche) angeordnet ist. Auf diese Weise ist die für die Lösung wirksame Oberfläche praktisch während der gesamten Dauer der Wirkstoffabgabe und dementsprechend auch die Wirkstoffabgabe selbst konstant.

Ein weiterer Vorteil des Einschlusses des Wirkstoffs W in ein durch eine flüssigkeitsdurchlässige Membrane abgeschlossenes Behältnis besteht darin, dass der Wirkstoff auf diese Weise nicht in einer speziellen Formulierung vorliegen muss, um die gewünschte zeitliche Charakteristik der Wirkstoffabgabe zu erzielen. Vielmehr genügt dazu bereits, wenn der Wirkstoff als Pulver oder Granulat in loser Schüttung vorliegt.

Wenn keine zeitliche Konstanz der Wirkstoffabgabe gefordert ist, kann das Wirkstoffbehältnis auch anders, beispielsweise etwa mit vertikaler Membrane, in der Staukammer angeordnet sein. Ueberhaupt kann das Wirkstoffbehältnis auch andere als die gezeigten Formen aufweisen, sofern nur wenigstens in Teil seiner Wandung aus einer flüssigkeitsdurchlässigen Membran besteht.

Analoges gilt für die Staukammer : Es ist lediglich erforderlich, dass sie an ihrem unteren Ende eine als Restriktion wirkende Abflussöffnung und in ihrem oberen Teil einen Ueberlauf aufweist und so angeordnet bzw. mit dem Einlass des Behälters verbunden ist, dass die eintretende Infusionsflüssigkeit auch in die Staukammer gelangt und aus ihr überlaufend zum Auslass des Behälters fliessen kann.

Als Kapillare kommt beispielsweise eine Nadel vom 0,4 mm Innendurchmesser infrage. Die flüssigkeitsdurchlässige Membran kann beispielsweise aus einem Cellulose-Ester mit 0,45 µm Porengrösse bestehen. Alle übrigen Teile der Wirkstoffabgabevorrichtung sind beispielsweise aus PVC.

## Patentansprüche

1. Wirkstoffabgabevorrichtung zum Einfügen in die Flüssigkeitsleitung eines parenteralen Applikationssystems mit einem einen oberen Einlass (41) und einen unteren Auslass (42) aufweisenden, von der Flüssigkeit zur durchströmenden Behälter (43) und mit einer darin befindlichen Anordnung zur Abgabe eines Wirkstoffs (W) in die durchströmende Flüssigkeit (F), wobei im Behälter (43) eine oben mit einem Ueberlauf (56) versehene, den Wirkstoff (W) enthaltende Staukammer (50) vorgesehen ist, die so im Strömungsweg zwischen Einlass (41) und Auslass (42) angeordnet ist, dass die über den Einlass (41) in den Behälter (43) eintretende Flüssigkeit (F) in die Staukammer (50) gelangt und aus dieser überlaufende Flüssigkeit an der Außenseite der Staukammer (50) zum Auslass (42) strömt, dadurch gekennzeichnet, dass die Staukammer (50) an ihrem unteren Ende eine als Restriktion ausgebildete Austrittsöffnung (53) aufweist, die in den unteren als Luftraum wirkenden Teil des Behälters (43) ausmündet, dass die Staukammer (50) derart im Behälter (43) angeordnet ist, dass die aus der Staukammer (50) überlaufende Flüssigkeit (F) im wesentlichen widerstandsfrei zum Auslass (42) des Behälters (43) strömen kann, und dass die Anordnung zur Abgabe des Wirkstoffs (W) in der Staukammer (50) als allseitig geschlossenes, den Wirkstoff (W) enthaltendes Behältnis (60) ausgebildet ist, dessen Wandung zumindest teilweise aus einer flüssigkeitsdurchlässigen Membrane (63) besteht.

2. Vorrichtung nach Anspruch 1, dadurch gekennzeichnet, dass die Staukammer (50) oben offen ausgebildet ist.

3. Vorrichtung nach Anspruch 1 oder 2, dadurch gekennzeichnet, dass die Austrittsöffnung der Staukammer (50) als Kapillare (63) ausgebildet ist.

4. Vorrichtung nach einem der Ansprüche 1 bis 3, dadurch gekennzeichnet, dass das Wirkstoffbehältnis (60) in der Staukammer (50) so angeordnet ist, dass es im wesentlichen allseitig von Flüssigkeit umgeben ist.

5. Vorrichtung nach einem der Ansprüche 1 bis 4, dadurch gekennzeichnet, dass das Wirkstoffbehältnis (60) als flache Mulde oder Napf ausgebildet ist, dessen im wesentlichen ebene Oeffnung mittels einer flüssigkeitsdurchlässigen Membran (63) verschlossen ist.

6. Vorrichtung nach Anspruch 5, dadurch gekennzeichnet, dass der die Mulde bzw. den Napf bildenden Teil (61) des Wirkstoffbehältnisses (60) aus einem flüssigkeitsundurchlässigen Material besteht.

7. Vorrichtung nach Anspruch 5 oder 6, dadurch gekennzeichnet, dass das Wirkstoffbehältnis (60) mit der Membrane (63) nach unten in der Staukammer (50) gehalten ist.

8. Vorrichtung nach einem der Ansprüche 1 bis 7, dadurch gekennzeichnet, dass die Staukammer (50) mittels Wandrippen (47) allseits im Abstand von den Innenwänden des Behälters (43) in diesem gehalten ist.

9. Parenterales Applikationssystem mit einem Vorratsbehälter (1) für die zu verabreichende Flüssigkeit, einer Applikationsnadel (6), einer die Nadel und den Vorratsbehälter verbindenden Flüssigkeitsleitung (7) und einer in die Flüssigkeitsleitung (7) eingefügten Wirkstoffabgabevorrichtung, dadurch gekennzeichnet, dass die Wirkstoffabgabevorrichtung (4) gemäss einem der vorstehenden Ansprüche ausgebildet ist.

## Claims

1. Dispensing device for an active substance for insertion in the liquid conduit of a parenteral application system with a container (43) through which the liquid is to flow and which has an upper inlet (41) and a lower outlet (42), and with an arrangement located therein for dispensing an active substance (W) into the liquid (F) flowing therethrough, a damming chamber (50) containing the active substance (W) being provided in the container (43) and being provided at its upper end with an overflow (56) and being so arranged in the flow path between the inlet (41) and the outlet (42) that the liquid (F) which enters the container (43) through the inlet (41) in the container (43) enters the damming chamber (50) and the liquid overflowing from this to the outside of the damming chamber (50) flows out through the outlet (42), characterised in that the damming chamber (50) has at its lower end a discharge opening (53) formed as a restriction, which opens into the lower part of the container (43) which serves as an air space, that the damming chamber (50) is arranged in the container (43) in such a way that the liquid (F) overflowing from the damming chamber (50) can flow to the outlet (42) of the container (43) substantially without resistance, and that the arrangement for dispensing the active substance (W) in the damming chamber is formed as a container (60) which is closed on all sides, which contains the active substance (W) and of which the wall consists at least partly of a liquid-permeable membrane (63).

2. Device according to Claim 1, characterised in that the damming chamber (50) is formed open at its upper end.

3. Device according to Claim 1 or 2, characterised in that the discharge opening of the damming chamber is formed as a capillary.

4. Device according to one of Claims 1 to 3, characterised in that the active substance container (60) is so arranged in the damming chamber (50) that it is substantially surrounded by liquid on all sides.

5. Device according to one of Claims 1 to 4, characterised in that the active substance container (60) is formed as a flat trough or bowl, the substantially planar opening of which is closed by a liquid-permeable membrane (63).

6. Device according to Claim 5, characterised in that the part (61) of the active substance container (60) forming the trough or bowl consists of a liquid-permeable material.

7. Device according to Claim 5 or 6, characterised in that the active substance container (60) is mounted in the damming chamber (50) with the membrane (63) underneath.

8. Device according to one of Claims 1 to 7, characterised in that the damming chamber (50) is mounted in the container (43) by means of wall ribs (47) by which it is spaced on all sides from the inner wall of the container.

9. Parenteral application system with a reservoir (1) for the liquid to be administered, an application needle (6), a liquid pipe connecting the needle and the reservoir and a dispensing device for active substance inserted in the liquid pipe, characterised in that the active substance dispensing device (4) is constructed according to one of the preceding claims.

**Revendications**

1. Dispositif de distribution de substance active, à insérer dans le conduit pour liquide d'un système d'administration parentérale, doté d'un récipient (43) devant être traversé par le liquide et présentant une entrée supérieure (41) et une sortie inférieure (42), ainsi que d'un dispositif, placé à l'intérieur du récipient, pour la distribution d'une substance active (W) dans le liquide (F) en écoulement, une chambre de retenue (50) contenant la substance active (W) et munie à sa partie supérieure d'un trop-plein (56) étant prévue dans le récipient (43) et disposée de telle façon dans le trajet d'écoulement entre l'entrée (41) et la sortie (42), que le liquide (F) admis dans le récipient (43) par l'entrée (41) parvient dans la chambre de retenue (50) et que le liquide débordant de cette chambre s'écoule le long de la face externe de la chambre de retenue (50) vers la sortie (42), caractérisé en ce que la chambre de retenue (50) présente à son extrémité inférieure un orifice de sortie (53) ayant fonction de restriction et débouchant dans la partie inférieure du récipient (43) faisant office d'espace libre, en ce que la chambre de retenue (50) est disposée dans le récipient (43) de sorte que le liquide (F) débordant de la chambre de retenue (50) puisse s'écouler essentiellement sans résistance vers la sortie (42) du récipient (43), et en ce que le dispositif de distribution de substance active (W) est conçu dans la chambre de retenue (50) comme un réservoir fermé de toutes parts contenant la substance active (W) et dont la paroi est constituée au moins partiellement d'une membrane (63) perméable au liquide.

2. Dispositif selon la revendication 1, caractérisé en ce que la chambre de retenue (50) est ouverte à sa partie supérieure.

3. Dispositif selon la revendication 1 ou 2, caractérisé en ce que l'orifice de sortie de la chambre de retenue (50) est conçu comme capillaire (53).

4. Dispositif selon l'une des revendications 1 à 3, caractérisé en ce que le réservoir (60) pour la substance active est disposé dans la chambre de retenue (50) de façon à être essentiellement entouré de toutes parts de liquide.

5. Dispositif selon l'une des revendications 1 à 4, caractérisé en ce que le réservoir (60) pour la substance active est en forme de calotte ou d'auge dont l'ouverture essentiellement plane est fermée par une membrane (63) perméable au liquide.

6. Dispositif selon la revendication 5, caractérisé en ce que la partie (61) du réservoir (60), formant la calotte ou auge du réservoir (60) pour la substance active, est formée d'un matériau imperméable.

7. Dispostif selon la revendication 5 ou 6, caractérisé en ce que le réservoir (60) pour la substance active est fixé, la membrane (63) tournée vers le bas, dans la chambre de retenue (50).

8. Dispositif selon l'une des revendications 1 à 7, caractérisé en ce que la chambre de retenue (50) est maintenue, au moyen de nervures de paroi (47), de tous côtés à distance des parois internes du récipient (43), dans ce dernier.

9. Système d'administration par voie parentérale, doté d'un réservoir de stockage (1) pour le liquide à administrer, d'une aiguille (6) pour l'injection, d'un conduit (7) pour liquide reliant l'aiguille et le réservoir de stockage, et d'un dispositif de distribution de substance active, inséré dans le conduit (7) pour liquide, caractérisé en ce que le dispositif (4) de distribution de substance active est conçu conformément à l'une des revendications précédentes.

**Fig.1**

**Fig.3**

**Fig.2**

**Fig.4**